# EUROPÄISCHE PATENTSCHRIFT

(11) **EP 1 572 844 B1**
(45) Veröffentlichungstag und Bekanntmachung des Hinweises auf die Patenterteilung: **22.04.2009**
(21) Anmeldenummer: 03767772.1
(22) Anmeldetag: 09.12.2003
(51) Int. Cl.: C11C 3/14, C07C 51/09, C07C 51/43, C07C 51/353, C07C 57/12

(54) **VERFAHREN ZUR HERSTELLUNG VON KONJUGIERTER LINOLSÄURE**
METHOD FOR PRODUCING CONJUGATED LINOLEIC ACID
PROCEDE POUR PRODUIRE DE L'ACIDE LINOLEIQUE CONJUGUE

(30) Priorität: 18.12.2002 DE 10259157
(43) Veröffentlichungstag der Anmeldung: 14.09.2005
(73) Patentinhaber: Cognis IP Management GmbH, 40589 Düsseldorf (DE)
(72) Erfinder: HORLACHER, Peter, 89287 Bellenberg (DE); RUF, Karl-Heinz, 87727 Babenhausen (DE); TIMMERMANN, Franz, 89257 Illertissen (DE); ADAMS, Wolfgang, 88074 Meckenbeuren (DE); KRIES, Rainer von, 89257 Illertissen (DE)
(86) Internationale Anmeldenummer: PCT/EP2003/013922
(87) Internationale Veröffentlichungsnummer: WO 2004/055142

(56) Entgegenhaltungen:
- EP-A- 0 950 410
- WO-A-01/40419

## Beschreibung

### Gebiet der Erfindung

Die Erfindung befindet sich auf dem Gebiet der Fettsäuren und betrifft ein neues Verfahren zur Herstellung von angereicherter konjugierter Linolsäure, die bei Temperaturen unterhalb von 10°C nicht zu Produkttrübungen führt.

### Stand der Technik

Herstellungsmethoden für die Gewinnung mehrfach ungesättigter Linolsäuren mit konjugierten Doppelbindungen, die unter der Bezeichnung "CLA" (conjugated linoleic acid) im Handel sind und vermehrt als Lebensmittelzusatzstoffe eingesetzt werden, sind aus der Literatur vielfach bekannt.

Die reine CLA wird in der Regel durch Verseifung linolsäurehaltiger Öle gewonnen. Nachteil dieser Verfahren ist der hohe Anteil unerwünschter Isomere und die geringe Ausbeute.
So beschreibt die Internationale Anmeldung WO 96/06605 A1 die Herstellung freier konjugierter Linolsäure ausgehend von Triglyceriden und Linolsäure, die in Ethylenglycol mit Kaliumhydroxid verseift und gleichzeitig isomerisiert werden. Die CLA wird nach erfolgter Neutralisation des Reaktionsgemisches mit Salzsäure anschließend mit einem organischen Lösungsmittel (Hexan) extrahiert und durch Waschen aufgereinigt.

Die Internationale Anmeldung WO 01/40419 A2 offenbart ein Verfahren zur Isomerisierung von linolsäurehaltigem Sonnenblumöl und anschließender Aufarbeitung.

In der Europäischen Patentschrift EP 0 839 897 B1 wird ein Verfahren offenbart bei dem Triglyceride mit Linolsäure einer alkalischen Isomerisation mit Kaliumhydroxid in Propylenglycol unterzogen werden. Die weitere Aufarbeitung erfolgt ebenfalls durch Neutralisation mit Salzsäure, Extraktion mit Hexan und Waschen mit 5 Gew. % NaCl-Lösung.

Ein ähnliches Verfahren zur Herstellung einer qualitativ hochwertigen CLA wird in der Europäischen Patentanmeldung EP 0 950 410 A1 beschrieben.

Bei der Herstellung der oben genannten konjugierten Linolsäure wurde von Saffloröl (Distelöl) oder Sonnenblumenöl ausgegangen. Ein großer Nachteil dieses Verfahrens besteht darin, dass einerseits der Gehalt an konjugierter Linolsäure durch den Linolsäuregehalt der Triglyceride bestimmt wird - es können üblicherweise nur Gehalte von 63-78% erhalten werden - und andererseits eine hohe Menge unerwünschter Abfallstoffe entsteht.
Ein weiterer Nachteil dieser Produkte ist der hohe Gehalt an gesättigten Fettsäuren (vor allem Palmitin- und Stearinsäure), der dazu führt, dass das Produkt bei tieferen Temperaturen (< 10°C) Niederschläge aufweist.
Um diese Nachteile zu beheben wird in der Regel der Gehalt an gesättigten Fettsäuren durch mehrmalige Destillation gesenkt. Dies führt jedoch zu deutlichen Ausbeuteverlusten, was aus wirtschaftlichen und ökologischen Gesichtspunkten unerwünscht ist

Die Aufgabe der vorliegenden Erfindung hat daher darin bestanden, ein Verfahren zur Herstellung von konjugierter Linolsäure zur Verfügung zu stellen, das sich durch eine hohe Rentabilität auszeichnet und in hoher Ausbeute zu einem Endprodukt guter Reinheit führt. Die hergestellte konjugierte Linolsäure soll sich durch eine gute Lagerstabilität auszeichnen und insbesondere bei tiefen Temperaturen nicht zu Ausfällungen neigen.

### Beschreibung der Erfindung

Gegenstand der Erfindung ist ein Verfahren zur Herstellung von konjugierter Linolsäure, bei dem man
(a) Linolsäuremethyl - und/oder - ethylester in Gegenwart von Alkalialkoholaten isomerisiert
(b) die dann konjugierten Linolsäuren aus dem Ester nach Verseifung freisetzt und
(c) die freien Linolsäuren einer Kristallisation unterzieht.

Überraschenderweise wurde gefunden, dass durch dieses Verfahren konjugierte Linolsäure mit hoher Reinheit und einer verbesserten Lagerungsstabilität bei tiefen Temperaturen in hoher Ausbeute gewonnen werden konnte.

Die Herstellung aus Linolsäuremethyl - und/oder - ethylestern bietet die Möglichkeit Produkte mit einem Gehalt von CLA > 78% herzustellen.
Das Einfügen des Kristallisationsschrittes führt zu einem wesentlich wirtschaftlicheren und ökonomischeren Verfahren als herkömmliche Prozessschritte wie eine Destillation, bei der es zu Ausbeuteverlusten in Größenordnungen von über 20 % kommt.
Durch fraktionierte Destillation der Linolsäuremethyl - und/oder - ethylester kann der Gehalt an unerwünschten Nebenprodukten wie Palmitinsäure soweit gesenkt werden, dass der Gehalt an konjugierter Linolsäure mehr als 78 Gew.% beträgt. Der Nachteil dieses Verfahrens liegt also zum einen in der Ausbeute der fraktionierten Destillation und zum anderen in unerwünschten Nebenprodukten (Methanol und/oder Ethanol, Restgehalte an Estern). Diese Nachteile konnten durch den Einsatz eines Kristallisationsschrittes anstelle der Destillation vermieden werden. Es wurde ein lagerungsstabiles Endprodukt gewonnen, dessen Lagerung bei tiefen Temperaturen - Temperaturen unterhalb von 10°C - nicht zu Ausfällungen führte.

### Konjugierte Linolsäure (CLA)

Unter konjugierter Linolsäure sind erfindungsgemäß vorzugsweise die Hauptisomeren 9cis,11trans Octadecadiensäure und 10trans,12cis sowie jedoch beliebige Isomerenmischungen, wie sie üblicherweise bei der Herstellung konjugierter Linolsäure anfallen. Die durch das erfindungsgemäße Verfahren hergestellten Rohstoffe sollen bereits einen hohen Anteil der bevorzugten Isomere enthalten.

### Linolsäuremethyl - und/oder - ethylester

Als Ausgangsstoffe für das erfindungsgemäße Verfahren werden konjugierte Linolsäuremethyl- und/oder -ethylester eingesetzt.

### Isomerisierung

Die Isomerisierung der Linolsäuremethyl - und/oder - ethylester wird mit Alkalialkoholaten unter Begasung mit Inertgas bei Temperaturen im Bereich von 90 bis 150°C, vorzugsweise 100 bis 130 °C und besonders bevorzugt 105 bis 125 °C durchgeführt.
In einer bevorzugten Ausführungsform werden Alkalialkoholate mit 1 bis 10 C-Atomen als Basen während der Isomerisierung verwendet, besonders bevorzugt werden Kaliummethanolat, Kaliumethanolat oder Kalium-t-butylat eingesetzt.

### Verseifung

Die Verseifung der isomerisierten Linolsäuremethyl - und/oder - ethylester mit wässrigen Alkalilaugen erfolgt bei Temperaturen im Bereich von 40 bis 90 °C, vorzugsweise 60 bis 80 °C und besonders bevorzugt 65 bis 75 °C. Sie wird bis zu einem Spaltgrad von 80 bis 100 Gew. %, vorzugsweise größer 98% durchgeführt.

### Freisetzung der CLA

Nach der Behandlung mit den Alkalilaugen liegt die CLA als Seife vor und wird durch Ansäuern mit Phosphorsäure oder Citronensäure freigesetzt. Danach erfolgt eine Phasentrennung bei der die wässrige Phase abgetrennt wird und die überstehende Lösung isoliert wird. Diese überstehende Lösung enthält das CLA und wird nochmals mit Wasser gewaschen und anschließend im Vakuum getrocknet.

### Kristallisation

Der für die ökonomische Durchführung wesentlichste Schritt im Verfahren ist die Kristallisation. Durch die Kristallisation wird sowohl der Gehalt an Palmitin- als auch an Stearinsäure gesenkt. Letztere ist für die Niederschlagsbildung bei tieferen Temperaturen mitentscheidend.

Die Kristallisation kann je nach vorliegender Qualität der konjugierten Linolsäure ein- oder mehrmals durchgeführt werden. Die Kristallisation wird bei Temperaturen < 10°C, bevorzugt < 6°C durchgeführt.

### Beispiel

### Herstellung von konjugierter Linolsäure

In einen beheizbaren Kolben wurden 1190 g Linolsäureethylester aus Safloröl vorgelegt und unter Rühren, Stickstoffbegasung und kontinuierlichem Abdestillieren von Ethanol, wurden bei einer Temperatur von 110°C 60 g Kaliumethanolat (32 Gew.%) zugefügt. Nach Zugabe von 190 g Wasser wurden bei einer Temperatur von 70°C 1070 g einer 25 Gew.%ige Kaliumhydroxid-Lösung zur Verseifung in den Kolben gepumpt.

Zur Freisetzung der freien Säure wurde der Ansatz mit 770 g Wasser versehen und bei 70°C 510 g Phosphorsäure (85 Gew.-%) hinzugegeben. Zur Phasentrennung wurde der Ansatz dann 30 Minuten bei 70°C stehengelassen. Nachdem die wässrige Phase abgetrennt worden war, wurde nochmals mit Wasser gewaschen und schließlich die organische Phase im Vakuum getrocknet.
Nach der Verseifung erfolgte die Kristallisation der freien konjugierten Linolsäuren.

### Kristallisation (Schritt c)

### Systembeschreibung:

| | |
|---|---|
| Steuerung: | PC mit Programm Wizcon |
| Heizung - Kühlung: | Julabo-Thermostat FP 50 |
| Kristallisiergefäß: | Kristallisator von DeSmet (Belgien) mit 17,8 Liter Gesamtinhalt |
| Rührer: | beinahe wandgängiger Ankerrührer, gesamte Gefäßhohe |
| Filterpresse: | kühlbare Filterpresse, Temperatur wie Badtemp., Kammer: Durchmesser 20 cm, Breite 1,8 cm, Inhalt 565 cm³ |

### Durchführung:

Die Fettsäure wird in den Kristallisator eingefüllt und auf 5°C abgekühlt.

**Tabelle 1: Ablauf der Kristallisation**

| Zeit | Bad-temp. | Produkt-temp. | Bemerkung |
|---|---|---|---|
| 0:42 | 3,0°C | 5,3°C | sehr gute Kristalle, Beginn Filtration |
| 0:44 | 3,0°C | 5,3°C | Filtratablaufbeginnt |
| 0:59 | 3,0°C | 5,4°C | 2 Liter Filtrat, 0,2 bar auf Behälter, 1, Probe entnehmen |
| 1:16 | 3,0°C | 5,5°C | 4 Liter Filtrat, 0,4 bar auf Behälter, 2. Probe entnehmen |
| 1:25 | 3,0°C | 5,6°C | Ende der Filtration, kurz mit N₂ ausblasen, dann nachpressen |

Die Ausbeute betrug nach der Kristallisation 91 %. Nach Abtrennung der Oligomeren, die destillativ erfolgt, betrug die Ausbeute 86 Gew.%
Auch bei der destillativen Aufkonzentrierung muß am Ende nochmals über Kopf destilliert werden, um oligomere "CLA"s abzutrennen, so dass ein erneuter Ausbeuteverlust auftritt..

### Analytik:

### GC-Methode: Silylierung der freien Fettsäuren mit anschließender Trennung auf Säule

**Tabelle 2: Zusammensetzung des Reaktionsproduktes vor und nach der Kristallisation.**

| ***FS-Spektrum*** | ***Ausgangsmaterial*** | ***nach Kristallisation*** |
|---|---|---|
| Fettsäure C12 | <0,1 | <0,1 |
| Fettsäure C14 | 0,1 | 0,1 |
| Fettsäure C15 | <0,1 | <0,1 |
| Fettsäure C16 | 6,6 | 4,4 |
| Fettsäure C16* | 0,1 | 0,1 |
| Fettsäure C17 | <0,1 | <0,1 |
| Fettsäure C18 | 2,5 | 1,3 |
| Fettsäure C18* | 13,5 | 14,1 |
| Fettsäure C18** | 0,9 | 0,9 |
| Fettsäure C20 | 0,3 | 0,2 |
| Fettsäure C20* | 0,2 | 0,2 |
| Fettsäure C22 | 0,2 | 0,1 |
| Fettsäure C22* | <0,1 | <0,1 |
| Fettsäure C24 | 0,1 | 0,1 |
| Fettsäure C24* | 0,1 | 0,1 |
| Fettsäure C18 konjug. | 72,6 | 75,7 |
| Fettsäure C18 konjug. Isomer 1 | 34,5 | 36,2 |
| Fettsäure C18 konjug. Isomer 2 | 35,6 | 37,2 |
| Fettsäure C18 konjug. Isomer 3 | 1,5 | 1,6 |
| Fettsäure C18 konjug. Isomer 4 | 0,8 | 0,7 |
| Summe Fettsäure frei | 97,3 | 97,4 |

Das Ausgangsmaterial (normales Endprodukt)entspricht der Herstellung der konjugierten Linolsäure ausgehend von Safloröl. Dieses wurde mit dem Endprodukt nach der Kristallisation verglichen. Das Beispiel zeigt, dass mit der Kristallisation eine Anreicherung des CLA-Gesamtgehaltes möglich ist.
Würde Beispiel aus Spalte 2 destilliert läge die Ausbeute bei vergleichbarer Qualität (CLA-Gehalt) bei etwa 79% im Vergleich zur Kristallisation mit 86%.

### Trübungsverhalten bei 8°C

Beide Produkte (Ausgangsmaterial ohne Kristallisation und kristallisiertes Material) wurden bei 8°C gelagert. Nur das kristallisierte Produkt blieb selbst nach mehrwöchiger Lagerung ohne Trübungserscheinungen.

## Patentansprüche

1. Verfahren zur Herstellung von konjugierter Linolsäure, bei dem man
(a) Linolsäuremethyl - und/oder - ethylester in Gegenwart von Alkalialkoholaten isomerisiert
(b) die dann konjugierten Linolsäuren aus dem Ester nach Verseifung freisetzt und
(c) die freien Linolsäuren einer Kristallisation unterzieht.

2. Verfahren nach Anspruch 1, **dadurch gekennzeichnet, dass** man die Kristallisation (c) bei Temperaturen unterhalb von 10°C durchführt.

3. Verfahren nach mindestens einem der Ansprüche 1 und/oder 2, **dadurch gekennzeichnet, dass** man den Kristallisationsschritt (c) einmal oder mehrfach durchführt.

## Claims

1. A process for the production of conjugated linoleic acid, in which
(a) linoleic acid methyl and/or ethyl esters are isomerized in the presence of alkali metal alcoholates,
(b) the then conjugated linoleic acids are released from the ester after saponification and
(c) the free linoleic acids are subjected to crystallization.

2. A process as claimed in Claim 1, **characterized in that** the crystallization (c) is carried out at temperatures below 10°C.

3. A process as claimed in at least one of Claims 1 and/or 2, **characterized in that** the crystallization step (c) is carried out one or more times.

## Revendications

1. Procédé de production d'acide linoléique conjugué, dans lequel
(a) des esters méthyliques et/ou éthyliques de l'acide linoléique sont isomérisés en présence d'alcoolates de métaux alcalins,
(b) les acides linoléiques ainsi conjugués sont libérés de l'ester après la saponification, et
(c) les acides linoléiques libres sont soumis à une cristallisation.

2. Procédé selon la revendication 1, **caractérisé en ce que** l'étape de cristallisation (c) est réalisée à des températures inférieures à 10°C.

3. Procédé selon au moins l'une des revendications 1 et/ou 2, **caractérisé en ce que** l'étape de cristallisation (c) est réalisée une ou plusieurs fois.
